# EUROPEAN PATENT SPECIFICATION

(11) **EP 0 506 172 B1**
(45) Date of publication and mention of the grant of the patent: **16.10.1996**
(21) Application number: 92200754.7
(22) Date of filing: 17.03.1992
(51) Int. Cl.: G01T 1/29, A61B 6/08, A61B 6/04

(54) **X-ray examination apparatus and console system suitable for use in such an X-ray examination apparatus**
Röntgenuntersuchungsapparat sowie Bedienungseinheit zur Verwendung in einem derartigen Röntgenuntersuchungsapparat
Appareil d'examen à rayons X et unité de commande convenant pour être utilisée dans un tel appareil d'examen

(30) Priority: 25.03.1991 EP 91200660
(43) Date of publication of application: 30.09.1992
(73) Proprietor: Philips Electronics N.V., 5621 BA Eindhoven (NL)
(72) Inventor: Kortstra, Jan Roelof Abel, NL-5656 AA Eindhoven (NL); van Es, Ludovicus Phillipus Cornelius, NL-5656 AA Eindhoven (NL); van Alst, Josephus Antonius Maria, NL-5656 AA Eindhoven (NL); Eerland, Karel Kornelis, NL-5656 AA Eindhoven (NL)
(74) Representative: Veenstra, Gustaaf

(56) References cited:
- EP-A- 0 066 008
- EP-A- 0 244 561
- EP-A- 0 365 737
- EP-A- 0 463 187
- DE-A- 3 137 516
- DE-B- 1 028 202
- US-A- 4 298 801

## Description

The invention relates to an X-ray examination apparatus, comprising a stand with an X-ray source and an X-ray detector which are arranged so as to face one another, absorption means which are situated near the X-ray source in order to limit an X-ray beam to be emitted by the X-ray source, a table for positioning an object between the X-ray source and the X-ray detector, a central control unit for applying control signals to the stand and the table, and a console system which is connected to the central control unit and which comprises controls for controlling the central control unit.

The invention also relates to a console system and a control console suitable for use in such an X-ray examination apparatus.

In a known X-ray examination apparatus of the kind set forth a U-shaped arm supports an X-ray image intensifier and the X-ray source. *Via* a parallelelogram construction, the U-shaped arm is connected to a vertical column which is slidable on rails on the floor. The stand enables rotation of the X-ray source and the X-ray image intensifier about an isocentre which is formed by the point of intersection of the central ray between the X-ray source and the image intensifier and a horizontal axis of rotation. During examination of a patient, the area of interest is arranged in the isocentre so that the position of this object in the image formed remains constant for different orientations of the X-ray source and the image intensifier. The patient can be moved to the correct position relative to the isocentre by displacement of the table in a horizontal or a vertical direction. During examination, a patient can be observed while being continuously exposed to a comparatively low dose, for example, 50 µRs⁻¹ (fluoroscopy), the X-ray image intensifier then forming a brightness-intensified image on its exit screen, which image is converted, using a television camera, into a video signal which is displayed on a monitor. Another imaging method is a method where a number of images is recorded on film, using a number of pulses of a comparatively high dose, for example from 50 to 100 µR. The high dose reduces a loss of contrast in the image due to quantum noise. Another imaging method is digital subtraction angiography. According to this method, an image formed without contrast medium is subtracted from an image formed while the blood vessels are filled with a contrast medium. As a result, an image is obtained which shows only the vascular system, the structures which would mask the vessels in a conventional image being absent because of the subtraction operation.

It is an object of the invention to provide an X-ray examination apparatus which can be simply and efficiently operated. It is also an object of the invention to provide an X-ray examination apparatus where the position of the console system can be optimized in respect of different examination circumstances.

To achieve this, an X-ray examination apparatus in accordance with the invention is characterized in that the console system comprises two independently displaceable control consoles with mutually different control functions, at least one control console comprising position adjusting means for adapting the control signals to different control console positions.

During radiological examinations and/or interventions for which use is made of an X-ray examination apparatus for fluoroscopy or the recording of images on film, a team of radiologists and/or surgeons and radiology assistants surrounds the patient positioned on the table. A division of the control tasks for the X-ray examination apparatus among the team members offers the advantage that different persons need attend only a smaller number of functions of the X-ray examination apparatus. As a result, more attention can be paid to the treatment of the patient. For example, it is advantageous to assign the task of positioning of the stand relative to the patient to an assistant whilst the surgeon can displace, at the appropriate instant, the isocentre relative to the patient by displacement of the table, followed by adjustment and initiation of the imaging process. Because the console system is subdivided into two separate parts, these parts can be arranged in the optimum positions for the surgeon as well as for the assistant, for example for the surgeon in the immediate vicinity of the patient and for the assistant in a position offering a clear view of the stand, the table and the patient. The position adjusting means ensure that from any position of the control consoles relative to the stand, manipulation of the controls leads to a logic effect for the user. This implies, for example that motions of the stand correspond as regards direction to directions of motion of the relevant control on the control console.

An embodiment of an X-ray examination apparatus in accordance with the invention is characterized in that a first control console comprises mainly controls for positioning the stand, the X-ray detector and the table, and also comprises the position adjusting means, the second control console comprising mainly controls for adjusting the absorption means and for adjusting imaging conditions.

Subdivision of the console system into two control consoles, one of which controls all displacements in space of the stand and the table, whilst the imaging is controlled *via* the second control console, offers the advantage that the person attending the imaging functions can fully concentrate on these functions from the for this person most favourable position. Displacement of the table top in the vertical and the horizontal direction is preferably controlled from a position offering a clear view of the surroundings, for example to prevent motions causing collisions between the table and the stand or to prevent unnecessary stress on catheters due to table displacement. Therefore, the control for table displacement is advantageously accommodated on the same control console as the control for the stand displacement, because a clear view of the patient surroundings is desirable for both functions.

A further embodiment of an X-ray examination apparatus in accordance with the invention is characterized in that each of the control consoles comprises an attachment mechanism for detachable attachment to the table.

The control consoles attached to the table are readily accessible during treatment of the patient and, contrary to control consoles which are arranged separately in space on a mobile stand, they do not restrict the free space around the X-ray examination apparatus.

A further embodiment of an X-ray examination apparatus in accordance with the invention is characterized in that the table comprises a table top and a rail which is arranged at least partly along an edge of the table top, the attachment mechanism of the control consoles comprising a movable contact portion which cooperates with the rail and also a grip which is connected to the contact portion in order to detach and secure the control consoles relative to the rail.

The control consoles can be secured to the rail at the desired side of the table. The contact portion can be released from the rail by squeezing the grip, the contact portion engaging the rail again after the grip has been released, thus keeping the control console unit in position. This offers the advantage that the console system can be readily displaced along the rail without having to be detached therefrom. This can be realised by sufficiently flexible clamping of the contact portion around the rail so that when a slight pressure is exerted on the console system in the upwards direction, the pressure exerted on the rail by its own weight is cancelled and the console system can be displaced.

A further embodiment of an X-ray examination apparatus in accordance with the invention is characterized in that the first control console comprises a table top displacement control which forms a grip for manual displacement of the table top in a horizontal direction and which can lock the table top in a horizontal direction.

The table top is usually secured to a carriage which is displaceable in a first horizontal direction. This carriage is secured to a further carriage which is displaceable in a second horizontal direction, extending perpendicularly to the first direction. For example, when the table displacement control is depressed, both carriages are unlocked after which the table top can be moved to the desired horizontal position by lateral movement of the table top displacement control. When the table top displacement control is depressed again, the carriages supporting the table top are locked again. The dual function of the table top displacement control enhances the ease of table top displacement.

A further embodiment of an X-ray examination apparatus in accordance with the invention is characterized in that the first control console comprises a stand control, motion of the stand control in a first direction causing rotation of the X-ray source and the X-ray detector about a first axis which extends parallel to a longitudinal direction of the table, whilst motion of the stand control in a second direction causes rotation of the X-ray source and the X-ray detector about a second axis which extends parallel to a width direction of the table.

Using the stand control which is displaceable, for example in two mutually perpendicular directions, the X-ray source and the X-ray detector can be rotated in two directions corresponding to the displacements of the control. As a result, stand control is easier and the lay-out of the control console is more orderly than when use is made of two separate controls which each control rotation of the X-ray source and the X-ray detector in a respective direction.

A further embodiment of an X-ray examination apparatus in accordance with the invention is characterized in that the first control console comprises the position adjusting means for adjusting a positive or negative direction of rotation of the stand about the axes in response to the motions of the stand control.

When the first control console is attached to a long side of the table, displacement of the stand control in the longitudinal direction of the patient causes the part of the stand situated over the table to be displaced in the direction corresponding to the direction of the stand control displacement. When the first control console is attached to the opposite side of the table, the displacement directions of the stand oppose the directions of the stand control displacements. The same holds for stand control displacements and stand displacements in a direction perpendicular to the longitudinal direction of the patient. This is avoided in that the control consoles are, for example electrically or optically connected to the table *via* the position adjusting means, so that the position of the control console relative to the table is transmitted to the central control unit. As a result, the central control unit controls the motors for the stand rotation in response to a change of position of the first control console so that the displacement directions of the stand control are interchanged relative to the stand displacements. Thus, the displacement directions of the part of the stand situated over the table remain the same as those of the stand control.

Another embodiment of an X-ray examination apparatus in accordance with the invention is characterized in that the first control console comprises the position adjusting means for interchanging the motions of the stand control relative to the axes, so that motion of the stand control in the first direction causes rotation of the X-ray source and the X-ray detector about the second axis whilst motion of the stand control in the second direction causes rotation of the X-ray source and the X-ray detector about the first axis.

When the first control console is arranged at a long table side, the displacement direction of the stand is the same as the displacement direction of the stand control. When the first control console is used along the short side of the table, the displacement directions of the stand control are rotated through 90° with respect to the displacement direction of the stand. When the rotation of the stand about the axes is interchanged relative to the displacement directions of the stand control, the stand will follow the directions of the stand control also when the first control console is arranged at a short side of the table.

A further embodiment of an X-ray examination apparatus in accordance with the invention is characterized in that the position adjusting means comprise a switch.

A switch whose position informs the central control unit as regards the position of the control console relative to the stand offers the advantage that the position of the control console relative to the stand is defined for the central control unit also when the control console is arranged in a position other than a position at the table side, for example on a mobile frame. Moreover, a switch is more reliable than, for example electric or optical contacts between the table and the control console because the operation of such contacts can be impeded by contamination.

A further embodiment of an X-ray examination apparatus in accordance with the invention is characterized in that the shape of the stand control deviates perceptibly from a shape of the table top displacement control.

The table top displacement control preferably has a shape and size substantially filling the palm of a hand, so that a lateral force can be manually exerted on the table top *via* the table top displacement control. Because displacement of the stand control merely causes a variation of an electric signal applied to the central control unit, the construction of this control is less rugged.

A further embodiment of an X-ray examination apparatus in accordance with the invention is characterized in that the first control console comprises a table height control for vertical displacement of the table top and a magnification control for displacement of the X-ray detector relative to the X-ray source, the table height control and the magnification control having a shape which perceptibly deviates from the shape of the stand control and the table top displacement control.

When a sheet is arranged over the table adjustment control, the detector control and the stand control, as may occur during operations for reasons of sterility, it is advantageous that the various functions of the controls can be recognized by touching. When the controls are visible, their function is preferably recognized by a pictogram provided near the controls.

Some embodiments of an X-ray examination apparatus and a console system in accordance with the invention will be described in detail hereinafter with reference to the accompanying drawing. Therein:
Fig. 1 shows an X-ray examination apparatus comprising a known console system,
Figs. 2a and 2b show a console system in accordance with the invention,
Fig. 3 shows a table provided with a rail for attachment of the console system,
Fig. 4 diagrammatically shows the attachment mechanism of the control consoles,
Fig. 5 diagrammatically shows movement directions of the stand for different positions of the first control console, and
Figs. 6a and 6b diagrammatically show stands which can be controlled by means of the console system.

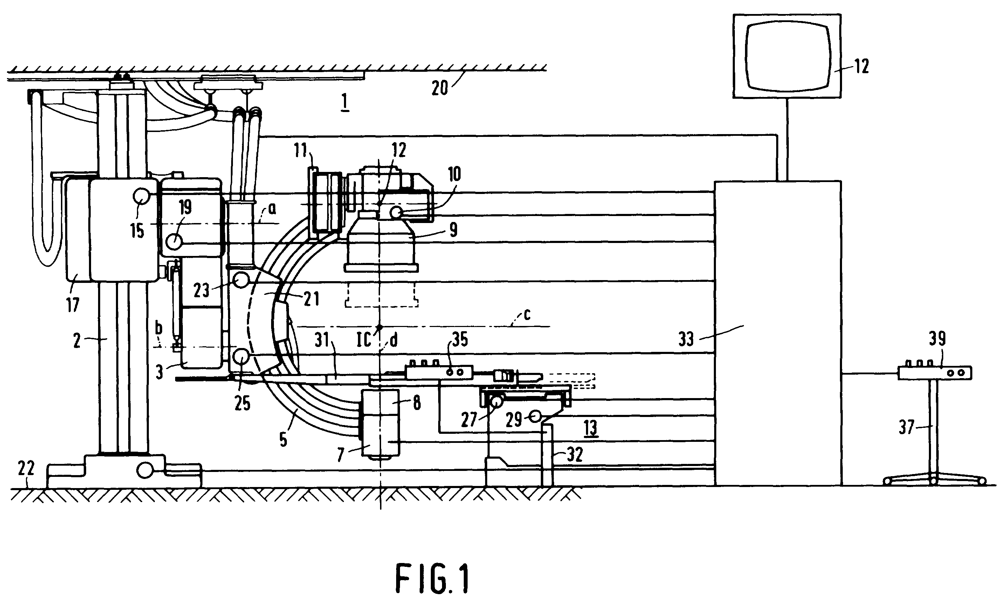

Fig. 1 shows a known X-ray examination apparatus, comprising a stand 1 which includes a vertical column 2 whereto a C-arm 5 is secured *via* an arm 3. The C-arm 5 supports an X-ray source 7 at a first end and an X-ray image intensifier tube 9 and a film changer 11 at a second end. The X-ray image intensifier tube 9, the film changer 11 and the X-ray source 7 are situated outside the plane of the C-arm 5. A patient can be arranged on a table 13 between the X-ray source 7 and the X-ray image intensifier tube 9. A carrier 17 which supports the arm 3 can be displaced along the column 2 by means of a motor 15. *Via* a motor 18, the column 2 is displaceable on rails arranged against the ceiling 20 and on the floor 22. A motor 19 provides rotation of the arm 3 about an axis a. A carrier 21 in which the C-arm 5 is secured and in which it can be displaced in a circumferential direction of the C-arm 5 under the control of a motor 23, rotates about an axis b under the control of a motor 23. A table top 31 of the table 13 is displaceable in a horizontal direction by a motor 27 and is displaceable in a vertical direction by a motor 29. The motors are controlled by a central control unit 33 which comprises a computer for the supply of control signals. *Via* an interface 32, the user of the X-ray examination apparatus can supply the central control unit with instructions by operation of controls of a console system 35 which is attached to the table 13 in known manner. In dependence on these instructions, the central control unit 33 applies control signals to a given motor, to the X-ray source 7 or to the X-ray image intensifier tube 9. Instructions can also be applied *via* a console system 39 mounted on a mobile stand 37.

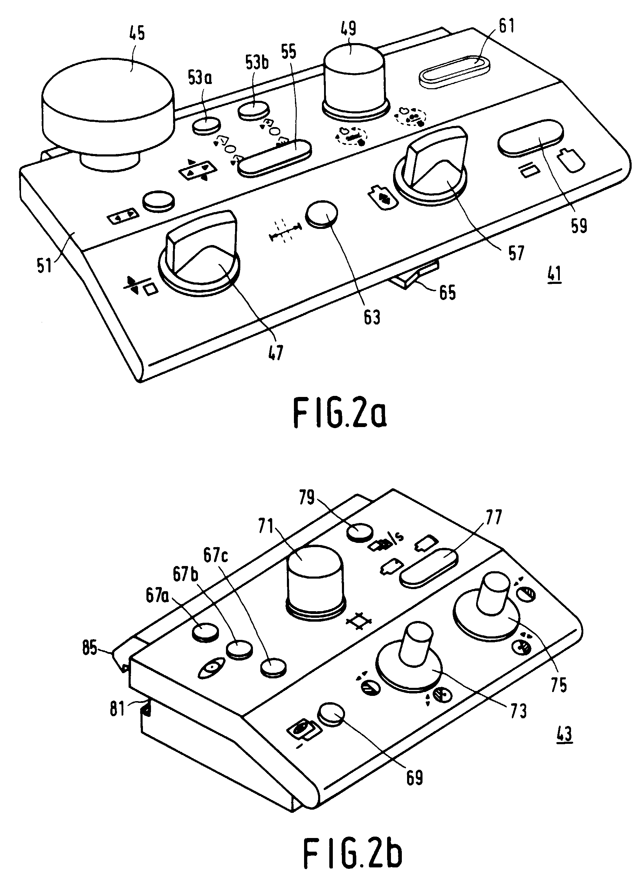

Figs. 2a and 2b show the console system 35 in accordance with the invention which comprises two control consoles 41 and 43. The control console 41 comprises the controls for positioning the stand 1 and the table 13, whilst the control console 43 comprises the controls for adjustment of the imaging and the positioning of the absorption means which include filters and beam-limiting diaphragms which are accommodated in a housing 8 in the vicinity of the X-ray source 7. The control console 41 comprises a table top displacement control 45, a table height control 47, a stand control 49, a control 51 for motor-driven table top displacement in a longitudinal direction of the table top 31, two storage controls 53a and 53b for the storage of a stand position in a memory of the central control unit 33, a recall control 55 for automatic adjustment of the stand 1 in the stand position stored in the memory of the central control unit 33, a magnification control 57 for adjusting the magnification of an image of an object by displacement of the X-ray image intensifier tube 9, a detector selection control 59, an emergency stop control 61, an override control 63 for overriding a limitation of stand positions of the stand 1 preprogrammed in the central control unit 33, and a switch 65 for interchanging the motions of the stand control 49 with respect to motions of the stand 1. The control console 43 comprises three controls 67a, 67b and 67c for selecting an image contrast on a monitor 12, a subtraction control 69, a diaphragm control 71 for positioning a beam-limiting diaphragm arranged in the housing 8, two wedge positioning controls 73 and 75 for positioning radiation-absorbing wedges in the X-ray beam for image harmonization, an image format control 77 for selecting the size of the detection face of the X-ray image intensifier tube 9, and a control 79 for adjusting the number of X-ray images formed per second. The functions of the controls of the control consoles 41 and 43 will be described in detail hereinafter.

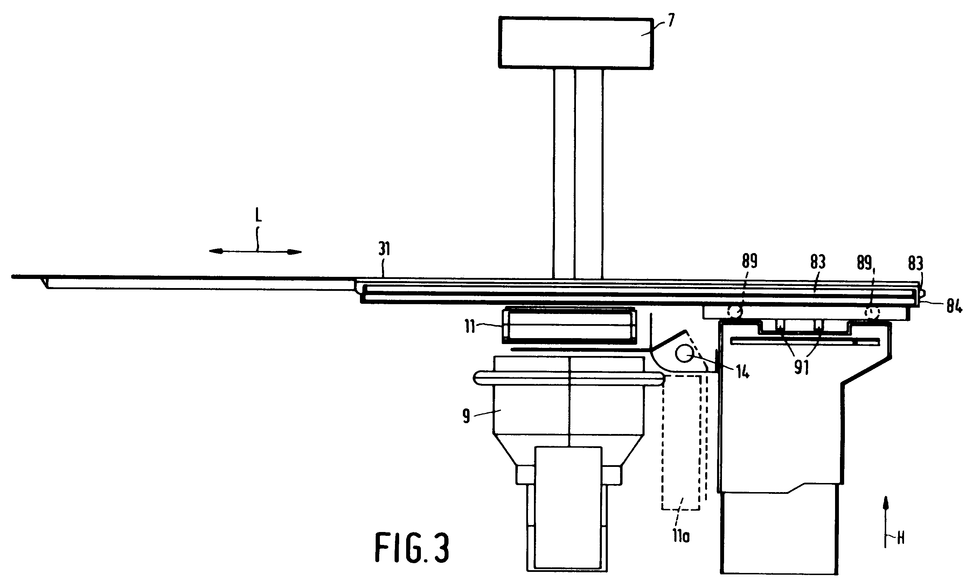

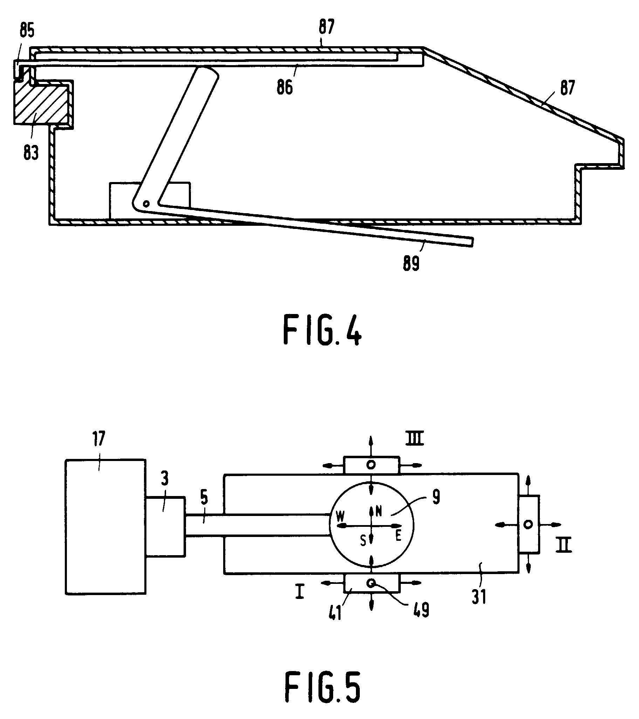

For the control console 43 Fig. 2b shows that at the rear it is provided with an attachment mechanism for attachment to the table 3. The attachment mechanism comprises a slot 81 which fits around a rail 83 secured to the table 13. Fig. 3 shows the table 13 with the rail 83. The rail 83 extends along two long sides and one short side 84 of the table 13. The control consoles 41 and 43 can be attached to the rails 83 by way of a movable contact portion 85 as shown in Fig. 4 which engages around an uppper edge of the rail 83. The contact portion 85 comprises, for example a resilient metal or plastics tongue 86 which is connected to the housing 87 of the control consoles 41, 43 at one end. When a pawl 89 is depressed against the resilience of the resilient tongue 86, the contact portion 85 is lifted so that the control console can be detached from the rail 83. Between the rails 83 and the walls of the slot 81 preferably a clearance of a few millimeters exists, so that the control consoles 41 and 43 can be slid along the rail 83 without it being necessary to detach the contact portion 85 from the rail 83.

The functions of some of the controls of the control consoles 41 and 43 will now be described in detail.

- The table top displacement control 45.
When the table top displacement control 45 is depressed once, the central control unit 33 receives an instruction to cancel blocking of the movement of the table top 31 in a horizontal direction. The table top 31 is manually displaceable in the longitudinal direction L *via* rollers 89 and is displaceable in a direction transversely of the plane of drawing *via* rollers 91. Upon displacement, the table top displacement control also serves as a grip. When the table top displacement control is depressed again, the table top position is locked by the central control unit 33, for example by blocking the rollers 89 and 91.

- The table height control 47.
When the table height control 47 is pushed forwards, the central control unit 33 activates the motor 29 so that the table top is lifted in the direction of the arrow H in Fig. 3. The table height is decreased when the table height control 47 is pulled backwards.

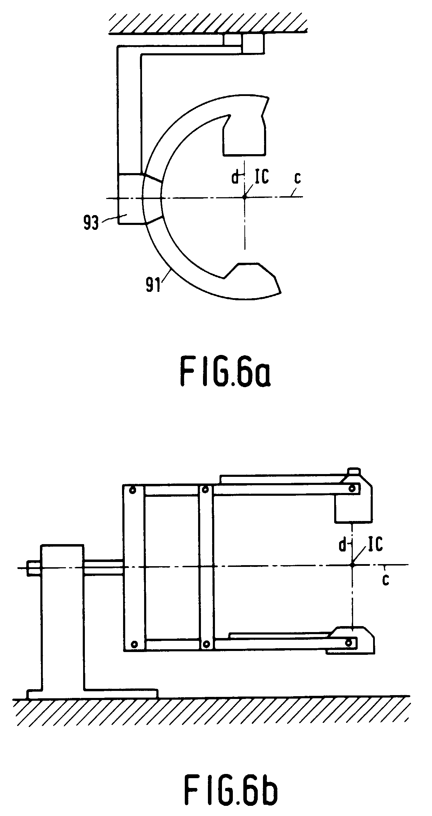

- The stand control 49.
The stand 1 can be displaced relative to the table 13 by movement of the stand control 49. When the control console unit 41 is attached to the long side I of the table 13 as shown in Fig. 5, movement of the stand control 49 to the front or the rear relative to the user causes a displacement of the X-ray image intensifier tube 9 (which can be replaced by the film changer 11) in the directions denoted by the arrows N and S, respectively. Displacement of the stand control 49 to the left and to the right causes a displacement of the X-ray image intensifier tube 9 in the directions denoted by the arrows W and E, respectively. The central control unit 33 then activates the motors 19, 23 and 25 so that a central ray d interconnecting the X-ray source 7 and the centre of the X-ray image intensifier tube 9 rotates about an axis c, extending in a longitudinal direction of the table top 31, and about a further axis which extends perpendicularly to the axis c and the central ray d. The point of intersection of the further axis and the axis c constitutes the isocentre IC which occupies the same position in all X-ray images.

When the control console 41 is arranged at the short side II of the table 13, motion of the stand control 49 in the forward and the backward direction relative to the user causes a motion of the X-ray image intensifier tube 9 in the directions denoted by the arrows W and E, respectively. Displacement of the stand control to the left and to the right relative to the user causes a motion of the X-ray image intensifier tube 9 in the direction of the arrows S and N, respectively.

When the control console 41 is arranged in the position III, the X-ray image intensifier tube 9 is displaced in the directions indicated by the arrows S, N, E and W, respectively, when relative to the user the stand control 49 is moved forwards, backwards, to the left and to the right, respectively. Using the control 65 on the control console 41, said control 65 having three positions, the position of the control console 41 relative to the stand is transmitted to the central control unit 33, so that the instructions generated *via* the stand control produce correct stand displacement.

Instead of using a control 65 for transmitting the position of the control console 41 to the control unit 33, use can also be made of an electrical contact between the control console 41 and the rail 83, the rails at different sides of the table then carrying different electric signals with a small voltage. A further alternative consists in providing the control console 41 with a photosensitive cell which receives a light signal which is generated at the table side and which differs for different table sides.

For stands having a geometry which deviates from that shown in Fig. 1, the control signals generated by the central control unit 33 in response to the instructions supplied by the stand control 49 deviate from those in the case shown in Fig. 1. For a stand as shown in Fig. 6a, the central ray d rotates about the isocentre IC by driving of the C-arm 91 in the carrier 93 or by rotation of the carrier 93 about the axis c. Both rotations are driven by a single motor as opposed to the stand shown in Fig. 1 where simultaneous driving of the motors 19, 23 and 25 is required for rotation of the central ray d about the isocentre. It will be evident that for rotation of the central ray d about an isocentre IC in the case of a parallelogram stand as shown in Fig. 6b, a control of the motors is required which deviates from that for stands as shown in the Figs. 1 and 6a. The control of a given type of stand can be realised by way of a single type of control console 41, 43 generating instructions which are applied to a central control unit 33 adapted to the relevant stand by programming.

- The magnification control 57.
When the magnification control 57 is moved forwards, the X-ray image intensifier tube 9 is displaced away from the X-ray source 7 along the central ray d. As a result, a magnification of the image increases. When the control 57 is moved backwards, the X-ray image intensifier tube 9 is moved towards the X-ray source 7, so that the magnification decreases.

- The detector selection control 59.
When the detector selection control is depressed, the central control unit 33 activates a motor 10 so that the X-ray image intensifier tube 9 and the film changer 11 rotate about an axis 12 extending transversely of the plane of drawing of Fig. 1 and the detection face of one of the detectors faces the X-ray source 7. When the film changer 11 is attached to the table 13 as shown in Fig. 3, the central control unit 33 activates a motor 14 so that the film changer 11 is moved from its position 11a in which it is folded against the table and which is denoted by an interrupted line in Fig. 3 to a position underneath the table top 31. When the X-ray image intensifier tube 9 is situated in the position of the film changer 11, the central control unit blocks the movement of the film changer 11.

- The image contrast controls 67a, 67b and 67c.
*Via* these controls an applied X-ray dose can be selected for which an acceptable image sharpness occurs.

- The subtraction control 69.
When this control is depressed, a monitor image is generated which consists of an X-ray image formed in the absence of contrast medium in the vessels and an X-ray image formed in the presence of a contrast medium in the vessels.

- The diaphragm control 71.
When the control 71 is moved forwards and backwards, a first pair of beam-limiting lead shutters, extending transversely of the X-ray beam emitted by the X-ray source 7, move towards and away from one another, respectively. Movement of the control 71 to the left and to the right causes a second pair of beam-limiting lead shutters whose edges extend perpendicularly to the edges of the first pair of lead shutters to move away from and towards one another, respectively. This produces a rectangular limitation of the X-ray image on the monitor, which limitation can be varied in dependence on the dimensions of the object to be irradiated. When the control 71 is depressed, the lead shutters return to a predetermined reset position.

- The wedge positioning controls 73 and 75.
When an X-ray image contains areas exhibiting a comparatively high radiation absorption in addition to areas exhibiting a comparatively low radiation absorption, for example the lungs and the heart, the details of sub-areas of lower contrast in the X-ray image is difficult to observe because of the great differences in brightness. When the X-ray beam is additionally attenuated by means of radiation-absorbing wedges in the areas of low radiation absorption, the dynamic range of the X-ray image is compressed so that a black level and a white level of the X-ray image can be adjusted to the darkest and brightest image values in the sub-area of interest. The dynamic range is increased within the sub-areas of interest. Movement of the controls 73 and 75 in the forward and the backward direction causes a radiation-absorbing wedge to rotate counterclock-wise and clock-wise, respectively, in the monitor image. Movement of the controls 73 and 75 to the left and to the right causes the radiation-absorbing wedges to move towards and away from the image centre, respectively. The actual motion of the radiation-absorbing wedges in the housing 8 depends on the stand position or on the angle of projection of the radiation-absorbing wedges in the X-ray image. On the basis of the stand position information, derived *inter alia* by angle encoders linked to the axes of rotation of the stand, correct driving of the radiation absorbing wedges is determined in the central control unit 33 so as to obtain a displacement in the X-ray image as indicated by the controls 73 and 75.

- The image format control 77.
For the imaging of organs in the abdomen of a patient, a large detection face of the X-ray image intensifier is desirable, for example 36 cm. For the imaging of smaller organs it is advantageous, *inter alia* because of scattered radiation, to utilize a smaller detection face of the X-ray image intensifier 9. When a smaller detection face is adjusted by means of the image format control 77, the positions of the beam-limiting lead shutters accommodated in the housing 8 are adapted to the new image format.

- The control 79 for adjusting the number of images formed per second.
For quickly moving organs pulsed operation of the X-ray source is necessary in order to avoid motional unsharpness; for pediatric examinations as many as 100 images per second may be required. The number of images formed per second is selected by depression of the control 79, it being possible to store two selected values in the memory of the control unit 33.

## Claims

1. An X-ray examination apparatus, comprising a stand (1) with an X-ray source (7) and an X-ray detector (9,11) are arranged so as to face one another, absorption means which are situated near the X-ray source (7) in order to limit an X-ray beam to be emitted by the X-ray source (7), a table (13) for positioning an object between the X-ray source (7) and the X-ray detector (9, 11), a central control unit (33) for applying control signals to the stand (1) and the table (13), and a console system (35, 39) which is connected to the central control unit (33) and which comprises controls for controlling the central control unit (33), characterized in that the console system (35, 39) comprises two independently displaceable control consoles (41, 43) with mutually different control functions, at least one control console comprising position adjusting means for adapting the control signals to different control console positions.

2. An X-ray examination apparatus as claimed in Claim 1, characterized in that a first control console (41) comprises mainly controls for positioning the stand (1), the X-ray detector (9, 11) and the table (13) and also comprises the position adjusting means, the second control console (43) comprising mainly controls for adjusting the absorption means and for adjusting imaging conditions.

3. An X-ray examination apparatus as claimed in Claim 1 or 2, characterized in that each of the control consoles (41, 43) comprises an attachment mechanism (81, 85) for detachable attachment to the table (13).

4. An X-ray examination apparatus as claimed in Claim 3, characterized in that the table (13) comprises a table top (31) and a rail (83) which is arranged at least partly along an edge of the table top (31), the attachment mechanism (81,85) of the control consoles (41,43) comprising a movable contact portion (85) which cooperates with the rail (83) and also a grip (89) which is connected to the contact portion (85) in order to detach and secure the control consoles (41,43) relative to the rail (83).

5. An X-ray examination apparatus as claimed in Claim 4, characterized in that the control consoles (41, 43) can be slid along the rail (83) without it being necessary to detach the contact portion (85).

6. An X-ray examination apparatus as claimed in Claim 3,4 or 5, characterized in that the first control console (41) comprises table top displacement control (45) which forms a grip for manual displacement of the table top (31) in a horizontal direction and which can lock the table top (31) in a horizontal direction.

7. An X-ray examination apparatus as claimed in any one of the Claims 2 to 6, characterized in that the first control console (41) comprises a stand control (49), motion of the stand control (49) in a first direction causing rotation of the X-ray source (7) and the X-ray detector (9, 11) about a first axis (c) which extends parallel to a longitudinal direction of the table (13), whilst motion of the stand control (49) in a second direction causes rotation of the X-ray source (7) and the X-ray detector (9, 11) about a second axis which extends parallel to a width direction of the table (13).

8. An X-ray examination apparatus as claimed in Claim 7, characterized in that the first control console (41) comprises the position adjusting means for adjusting a positive or negative direction of rotation of the stand (1) about the axes in response to the motions of the stand control (49).

9. An X-ray examination apparatus as claimed in Claim 7 or 8, characterized in that the first control console (41) comprises the position adjusting means for interchanging the motions of the stand control (49) relative to the axes, so that motion of the stand control (49) in the first direction causes rotation of the X-ray source (7) and the X-ray detector (9, 11) about the second axis, whilst motion of the stand control (49) in the second direction causes rotation of the X-ray source (7) and the X-ray detector (9, 11) about the first axis.

10. An X-ray examination apparatus as claimed in Claim 8 or 9, characterized in that the position adjusting means comprise a switch (65).

11. An X-ray examination apparatus as claimed in Claim 6 and 7, characterized in that the shape of the stand control (49) deviates perceptibly from a shape of the table top displacement control (45).

12. An X-ray examination apparatus as claimed in Claim 6 and 7, or Claim 11, characterized in that the first control console (41) comprises a table height control (47) for vertical displacement of the table top (31) and a magnification control (57) for displacement of the X-ray detector (9, 11) relative to the X-ray source (7), the table height control (47) and the magnification control (57) having a shape which perceptibly deviates from the shape of the stand control (49) and the table top displacement control (45).

13. An X-ray examination apparatus as claimed in any one of the preceding Claims, characterized in that the functions of the controls are stated on the control consoles (41, 43) by way of a pictogram.

14. A console system suitable for use in an X-ray examination apparatus as claimed in any one of the preceding Claims.

## Patentansprüche

1. Röntgenuntersuchungsapparat mit einem Ständer (1) mit einer Röntgenquelle (7) und einem Röntgendetektor (9, 11), die derart angeordnet sind, daß sie einander zugewandt sind, mit Absorptionsmitteln, die in der Nähe der Röntgenquelle (7) zum Begrenzen eines Röntgen strahls beim Ausstrahlen aus der Röntgenquelle (7) angeordnet sind, mit einem Tisch (13) zum Positionieren eines Gegenstands zwischen der Röntgenquelle (7) und dem Röntgendetektor (9, 11), mit einer Zentraleinheit (33) zum Zuführen von Steuersignalen nach dem Ständer (1) und dem Tisch (13), und mit einem Kontrollgerät (35, 39), das mit der Zentraleinheit (33) verbunden ist und Steuerungen zum Steuern der Zentraleinheit (33) enthält, dadurch gekennzeichnet, daß das Kontrollgerät (35, 39) zwei unabhängig voneinander verschiebbare Steuerpulte (41, 43) mit gegenseitig verschiedenen Steueraufgaben enthält, mit wenigstens einem Steuerpult mit Positionseinstellmitteln zum Anpassen der Steuersignale an verschiedene Steuerpultpositionen.

2. Röntgenuntersuchungsapparat nach Anspruch 1, dadurch gekennzeichnet, daß ein erstes Steuerpult (41) hauptsächlich Steuerungen zum Positionieren des Ständers (1), des Röntgendetektors (9, 11) und des Tisches (13) enthält und ebenfalls die Position sein stellmittel umfaßt, wobei das zweite Steuerpult (43) hauptsächlich Steuerungen zum Einstellen der Absorptionsmittel und zum Einstellen der Abbildungsbedingungen enthält.

3. Röntgenuntersuchungsapparat nach Anspruch 1 oder 2, dadurch gekennzeichnet, daß jedes der Steuerpulte (41, 43) eine Befestigungsvorrichtung (81, 85) zum lösbaren Befestigen am Tisch (13) enthält.

4. Röntgenuntersuchungsapparat nach Anspruch 3, dadurch gekennzeichnet, daß der Tisch (13) ein Tischblatt (31) und eine Schiene (83) enthält, die wenigstens teilweise entlang einem Rand des Tischblattes (31) angeordnet ist, wobei die Befestigungsvorrichtung (81, 85) der Steuerpulte (41, 43) einen beweglichen Kontaktanteil (85) enthält, der mit der Schiene (83) zusammenarbeitet, und ebenfalls einen Handgriff (89) umfaßt, der mit dem Kontaktanteil (85) zum Lösen und Befestigen der Steuerpulte (41, 43) in bezug auf die Schiene (83) verbunden ist.

5. Röntgenuntersuchungsapparat nach Anspruch 4, dadurch gekennzeichnet, daß die Steuerpulte (41, 43) auf der Schiene (83) verschiebbar sind, ohne daß sie vom Kontaktanteil (85) gelöst werden müssen.

6. Röntgenuntersuchungsapparat nach Anspruch 3, 4 oder 5, dadurch gekennzeichnet, daß das erste Steuerpult (41) eine Tischblattverschiebungssteuerung (45) enthält, die einen Handgriff zum handbetätigten Verschieben des Tischblattes (31) in einer horizontalen Richtung bildet und das Tischblatt (31) in einer horizontalen Richtung verriegeln kann.

7. Röntgenuntersuchungsapparat nach einem der Ansprüche 2 bis 6, dadurch gekennzeichnet, daß das erste Steuerpult (41) eine Ständersteuerung (49) enthält, wobei eine Bewegung der Ständersteuerung (49) in einer ersten Richtung eine Drehung der Röntgenquelle (7) und des Röntgendetektors (9, 11) um eine erste Achse (c) bewirkt, die sich parallel zu einer Längsrichtung des Tisches (13) erstreckt, während eine Bewegung der Ständersteuerung (49) in einer zweiten Richtung eine Drehung der Röntgenquelle (7) und des Röntgendetektors (9, 11) um eine zweite Achse bewirkt, die sich parallel zu einer breiten Richtung des Tisches (13) erstreckt.

8. Röntgenuntersuchungsapparat nach Anspruch 7, dadurch gekennzeichnet, daß das erste Steuerpult (41) die Positionseinstellmittel zum Einstellen eines positiven oder negativen Drehrichtung des Ständers (1) um die Achsen in Beantwortung der Bewegungen der Ständersteuerung (49) enthält.

9. Röntgenuntersuchungsapparat nach Anspruch 7 oder 8, dadurch gekennzeichnet, daß das erste Steuerpult (41) die Positionseinstellmittel zum Wechseln der Bewegungen der Ständersteuerung (49) in bezug auf die Achsen enthält, so daß eine Bewegung der Ständersteuerung (49) in der ersten Richtung eine Drehung der Röntgenquelle (7) und des Röntgendetektors (9, 11) um die zweite Achse bewirkt, während eine Bewegung der Ständersteuerung (49) in der zweiten Richtung eine Drehung der Röntgenquelle (7) und des Röntgendetektors (9, 11) um die erste Achse bewirkt.

10. Röntgenuntersuchungsapparat nach Anspruch 8 oder 9, dadurch gekennzeichnet, daß die Positionseinstellmittel einen Schalter (65) enthalten.

11. Röntgenuntersuchungsapparat nach Anspruch 6 und 7, dadurch gekennzeichnet, daß die Form der Ständersteuerung (49) wahrnehmbar abweicht von einer Form der Tischblattverschiebungssteuerung (45).

12. Röntgenuntersuchungsapparat nach Anspruch 6 und 7, oder nach Anspruch 11, dadurch gekennzeichnet, daß das erste Steuerpult (41) eine Tischhöhensteuerung (47) für die vertikale Verschiebung des Tischblattes (31) und eine Verstärkungssteuerung (57) zur Verschiebung des Röntgendetektors (9, 11) in bezug auf die Röntgenquelle (7) enthält, wobei die Tischhöhensteuerung (47) und die Verstärkungssteuerung (57) eine Form haben, die wahrnehmbar abweichen von der Form der Ständersteuerung (49) und der Tischblattverschiebungssteuerung (45).

13. Röntgenuntersuchungsapparat nach einem oder mehreren der vorangehenden Ansprüche, dadurch gekennzeichnet, daß die Aufgaben der steuerungen auf den Steuerpulten (41, 43) mittels eines Pictogramms angegeben sind.

14. Kontrollgerät zur Verwendung in einem Röntgenuntersuchungsapparat nach einem oder mehreren der vorangehenden Ansprüche.

## Revendications

1. Appareil d'examen aux rayons X, comprenant un statif (1) avec une source de rayons X (7) et un détecteur de rayons X (9, 11) qui sont disposés de manière à être face l'un à l'autre, des moyens d'absorption qui sont situés à proximité de la source de rayons X (7) afin de limiter un faisceau de rayons X émis par la source de rayons X (7), une table (13) pour positionner un objet entre la source de rayons X (7) et le détecteur de rayons X (9, 11), une unité centrale de commande (33) pour appliquer des signaux de commande au statif (1) et à la table (13), et une système de pupitres de commande (35, 39) qui est connecté à l'unité centrale de commande (33) et qui comprend des commandes pour commander l'unité centrale de commande (33), caractérisé en ce que le système de pupitres de commande (35, 39) comprend deux pupitres de commande (41, 43) déplaçables de manière indépendante, avec des fonctions de commande mutuellement différentes, au moins un pupitre de commande comprenant des moyens d'ajustement de la position pour adapter les signaux de commande à différentes positions du pupitre de commande.

2. Appareil d'examen aux rayons X suivant la revendication 1, caractérisé en ce qu'un premier pupitre de commande (41) comprend principalement des commandes pour positionner le statif (1), le détecteur de rayons X (9, 11) et la table (13) et comprend également les moyens d'ajustement de la position, le deuxième pupitre de commande (43) comprenant principalement des commandes pour régler les moyens d'absorption et pour régler les conditions de prise d'images.

3. Appareil d'examen aux rayons X suivant la revendication 1 ou 2, caractérisé en ce que chacun des pupitres de commande (41, 43) comprend un mécanisme de fixation (81, 85) permettant de le fixer de manière détachable à la table (13).

4. Appareil d'examen aux rayons X suivant la revendication 3, caractérisé en ce que la table (13) comprend un plateau (31) et un rail (83) qui est disposé au moins en partie le long d'un bord du plateau (31) de la table, le mécanisme de fixation (81, 85) des pupitres de commande (41, 43) comprenant une partie de contact mobile (85) qui coopère avec le rail (83) et également, une manette (89) qui est reliée à la partie de contact (85) de manière à détacher et fixer les pupitres de commande (41, 43) par rapport au rail (83).

5. Appareil d'examen aux rayons X suivant la revendication 4, caractérisé en ce que les pupitres de commande (41, 43) peuvent coulisser le long du rail (83) sans devoir nécessairement être détachés de la partie de contact (85).

6. Appareil d'examen aux rayons X suivant la revendication 3, 4 ou 5, caractérisé en ce que le premier pupitre de commande (41) comprend une commande de déplacement de plateau de table (45) qui forme une manette pour le déplacement manuel du plateau de table (31) dans une direction horizontale et qui peut verrouiller le plateau (31) de la table dans une direction horizontale.

7. Appareil d'examen aux rayons X suivant l'une quelconque des revendications 2 à 6, caractérisé en ce que le premier pupitre de commande (41) comprend une commande de statif (49), le déplacement de la commande de statif (49) dans une première direction provoquant la rotation de la source de rayons X (7) et du détecteur de rayons X (9, 11) autour d'un premier axe (c) qui s'étend parallèlement à une direction longitudinale de la table (13), tandis que le déplacement de la commande de statif (49) dans une deuxième direction provoque la rotation de la source de rayons X (7) et du détecteur de rayons X (9, 11) autour d'un deuxième axe qui s'étend parallèlement à une direction transversale de la table (13).

8. Appareil d'examen aux rayons X suivant la revendication 7, caractérisé en ce que le premier pupitre de commande (41) comprend les moyens d'ajustement de la position permettant de régler un sens positif ou négatif pour la rotation du statif (1) autour des axes en réaction aux déplacements de la commande de statif (49).

9. Appareil d'examen aux rayons X suivant la revendication 7 ou 8, caractérisé en ce que le premier pupitre de commande (41) comprend les moyens d'ajustement de la position permettant de permuter les déplacements de la commande de statif (49) par rapport aux axes, de telle sorte que le déplacement de la commande de statif (49) dans la première direction provoque la rotation de la source de rayons X (7) 0 et du détecteur de rayons X (9, 11) autour du deuxième axe, tandis que le déplacement de la commande de statif (49) dans la deuxième direction provoque la rotation de la source de rayons X (7) et du détecteur de rayons X (9, 11) autour du premier axe.

10. Appareil d'examen aux rayons X suivant la revendication 8 ou 9, caractérisé en ce que les moyens d'ajustement de la position comprennent un interrupteur (65).

11. Appareil d'examen aux rayons X suivant les revendications 6 et 7, caractérisé en ce que la forme de la commande de statif (49) s'écarte de manière perceptible d'une forme de la commande de déplacement de plateau de table (45).

12. Appareil d'examen aux rayons X suivant les revendications 6 et 7, ou la revendication 11, caractérisé en ce que le premier pupitre de commande (41) comprend une commande de hauteur de table (47) pour le déplacement vertical du plateau (31) de la table et une commande du grossissement (57) pour le déplacement du détecteur de rayons X (9, 11) par rapport à la source de rayons X (7), la commande de hauteur de table (47) et la commande du grossissement (57) ayant une forme qui diffère de manière perceptible de la forme de la commande de statif (49) et de la commande de déplacement du plateau de table (45).

13. Appareil d'examen aux rayons X suivant l'une quelconque des revendications précédentes, caractérisé en ce que les fonctions des commandes sont spécifiées sur les pupitres de commande (41, 43) par un pictogramme.

14. Système de pupitres de commande propre à être utilisé dans un appareil d'examen aux rayons X suivant l'une quelconque des revendications précédentes.
